# EUROPEAN PATENT SPECIFICATION

(11) **EP 2 866 029 B1**
(45) Date of publication and mention of the grant of the patent: **03.05.2017**
(21) Application number: 13806851.5
(22) Date of filing: 04.03.2013
(51) Int. Cl.: G01N 33/48, G01N 33/574

(54) **METHOD FOR PREPARING SAMPLE CONTAINING PANCREATIC JUICE COMPONENT, AND KIT FOR STORING BIOLOGICAL SAMPLE CONTAINING PANCREATIC JUICE COMPONENT AT ROOM TEMPERATURE**
VERFAHREN ZUR HERSTELLUNG EINER PROBE MIT EINER PANKREASSAFTKOMPONENTE SOWIE KIT ZUR LAGERUNG DER PROBE MIT EINER PANKREASSAFTKOMPONENTE BEI RAUMTEMPERATUR
PROCÉDÉ POUR LA PRÉPARATION D'UN ÉCHANTILLON CONTENANT UN COMPOSANT DE SUC PANCRÉATIQUE, ET TROUSSE POUR LE STOCKAGE D'UN ÉCHANTILLON BIOLOGIQUE CONTENANT UN COMPOSANT DE SUC PANCRÉATIQUE À LA TEMPÉRATURE AMBIANTE

(30) Priority: 22.06.2012 JP 2012140796
(43) Date of publication of application: 29.04.2015
(73) Proprietor: Olympus Corporation, Tokyo 192-8507 (JP)
(72) Inventor: KATAOKA, Rie, Tokyo 192-8507 (JP); MORIYA, Nao, Tokyo 192-8507 (JP); SANUKI, Hiromi, Tokyo 192-8507 (JP)
(74) Representative: Schicker, Silvia
(86) International application number: PCT/JP2013/055828
(87) International publication number: WO 2013/190867

(56) References cited:
- EP-A1- 2 757 151
- WO-A1-2012/137832
- WO-A1-2013/038981
- JP-A- 2010 025 770
- JP-A- 2011 047 802
- "The complete Guide for Protease Inhibition", , 1 August 2007 (2007-08-01), pages 1-20, XP055224046, Retrieved from the Internet: URL:www.roche-applied-science.com [retrieved on 2015-10-28]
- SABINE WANDSCHNEIDER ET AL: "Autoimmune pancreatic disease: Preparation of pancreatic juice for proteome analysis", ELECTROPHORESIS, vol. 22, no. 20, 14 December 2001 (2001-12-14), pages 4383-4390, XP055147890, DE ISSN: 0173-0835, DOI: 10.1002/1522-2683(200112)22:20<4383::AID-E LPS4383>3.0.CO;2-Z
- SARFATI P ET AL: "Evidence of a new serine protease in the rat pure pancreatic juice that degrades somatostatin", LIFE SCIENCES, PERGAMON PRESS, OXFORD, GB, vol. 47, no. 12, 1 January 1990 (1990-01-01), pages 1043-1049, XP025556204, ISSN: 0024-3205, DOI: 10.1016/0024-3205(90)90477-9 [retrieved on 1990-01-01]
- Toshifumi Furui ET AL: "Protein degradation in human pure pancreatic juice analyzed by two-dimensional gel electrophoresis", Electrophoresis, 1 January 1996 (1996-01-01), pages 797-802, XP055223510, Retrieved from the Internet: URL:http://onlinelibrary.wiley.com/doi/10. 1002/elps.1150170430/pdf [retrieved on 2015-10-26]
- STEINER J M ET AL: "PURIFICATION OF CLASSICAL PANCREATIC LIPASE FROM DOG PANCREAS", BIOCHEMIE, XX, XX, vol. 84, no. 12, 1 December 2002 (2002-12-01), pages 1243-1251, XP007900681,
- "Protease Inhibitor Panel Product information", , 8 September 2005 (2005-09-08), XP055224050, Retrieved from the Internet: URL:www.sigma-aldrich.com [retrieved on 2015-10-28]

## Description

### Technical Field

The present invention relates to a method for preparing a storage sample capable of stably storing S100P contained in pancreatic juice at room temperature until it is subjected to a test, and a kit suited for this method and for storing a pancreatic juice component-containing biological sample at room temperature, as further defined in the claims.

### Background Art

The body fluid is an important biological sample from which the state of organs can be known. In particular, pancreatic juice is an important biological sample useful for early detection of a pancreatic cancer which cannot be found early and is said to have a poor prognosis. It has been used for cytology, bicarbonate measurement, bacteria test, test of a marker made of a protein, nucleic acid, or the like, and the like. In recent years, analysis of a protein in pancreatic juice, for example, search of novel markers for, for example, pancreas-related diseases such as pancreatic cancer has been performed actively.

In particular, it has been reported that very frequent expression of S100P which is a member of an S100 protein family and is a receptor for advanced glycation end products (RAGE) in pancreatic cancer is confirmed and it is involved in growth and invasion of pancreatic cancer. For example, it has been reported in Non-patent Document 1 that S100P is useful as an early marker of pancreatic cancer and analysis of a S100P concentration in pancreatic juice is useful for discriminating between tumor and chronic pancreatitis. It has been reported in Non-patent Document 2 that expression of S100P is not observed in a normal pancreatic ductal epithelium, but expression of S100P is observed in all the specimens from intraductal papillary mucinous tumor cells. This means that S100P is useful as a marker for pancreatic cancer and measurement of an S100P amount in pancreatic juice is expected to be useful for early detection of pancreatic cancer, diagnosis of the stage of it, or the like.

On the other hand, pancreatic juice is a digestive juice so that it contains various digestive enzymes. It is known that these enzymes are present in inactive form in the pancreas, but are activated after excreted into the duodenum. A cascade degradation reaction of the digestive enzymes in pancreatic juice is accelerated by small intestinal enterokinase secreted from duodenal epithelial cells. It is known that trypsinogen contained in pancreatic juice is activated into trypsin by the small intestinal enterokinase and triggered by this trypsin, various digestive enzymes (proteases) such as chymotrypsinogen and proesterase are activated. By these various proteases thus activated, various biological molecules such as proteins, nucleic acids, lipids, and cells contained in pancreatic juice are inevitably degraded or modified after excreted into the duodenum. When the pancreatic juice excreted into the duodenum is used as a biological sample and the amount of S100P in this biological sample is determined, there is a fear that the amount cannot be measured correctly due to the influence of proteases on S100P in the biological sample. It is therefore important to know how to inhibit the activity of proteases in the pancreatic juice excreted into the duodenum.

It is presumed that since the protease activity generally depends on temperatures, degradation or modification of S100P during storage term can be suppressed to some extent by storing, in frozen form, the pancreatic juice or duodenal juice collected from a living body until it is subjected to the test of S100P. When the test of S100P is made as a clinical test, however, it is desirable to store the pancreatic juice or duodenal juice collected from a living body preferably at room temperature until it is subjected to the test in view that all the medical institutions or testing institutions do not always have sufficient storage equipment for specimens. Even when the pancreatic juice or duodenal juice is stored at room temperature, in order to stably store S100P contained in these specimens, the activity of proteases derived from the pancreatic juice or duodenal juice should be inhibited sufficiently even in solution form at room temperature.

As a method of storing a biological component-containing solution at room temperature, there is, for example, a method of, in performing immunoassay such as enzyme immunoassay or radio immunoassay in the field of clinical tests, adding casein or whey protein to a sample containing an antigen as an analyte and thereby preventing deactivation of the antigen in the sample in solution form and stably storing it (refer to, for example, Patent Document 1). As a method of stably storing a body fluid at room temperature, there is a method of immersing the target body fluid in a detection tool having a protease inhibitor supported on a porous material and then drying (refer to, for example, Patent Document 2).

### Citation List

### Patent Documents

Patent Document 1: Japanese Patent Application, First Publication No. Hei 8-5634
Patent Document 2: Japanese Patent Application, First Publication No. 2003-270237

### Non-Patent Documents

Non-patent Document 1: Ohuchida, and nine others, Clinical Cancer Research, 2006, Vol. 12, No. 18, pp. 5411-5416.
Non-patent Document 2: Nakata and nine others, Human Pathology, 2010, Vol. 41, pp. 824-831.

### Summary of the Invention

### Problems to be solved by the Invention

The method described in Patent Document 1 is effective for a sample containing a very small amount of foreign substances such as proteases, for example, a standard solution or the like to be used for immunoassay, but its effect for a sample originally containing a large amount of proteases, for example, pancreatic juice or duodenal juice cannot be expected so much.

The method described in Patent Document 2 stores specimens in the dry state so that it is effective to some extent when a test object is a substance that does not relatively undergo modification easily by drying treatment, for example, peptides; but proteins including S100P may be modified because their steric structure is affected by drying treatment. Moreover, Patent Document 2 describes many protease inhibitors that can be supported on a porous material but it does not disclose combinations of protease inhibitors optimum for suppressing degradation of S100P in pancreatic or duodenal juice. Even when pancreatic juice or the like is stored by using the method described in Patent Document 2 while omitting the drying treatment, a sufficient effect for storing S100P at room temperature cannot be produced.

An object of the present invention is to provide a method for preparing a pancreatic juice component-containing sample capable of stably storing, particularly S100P, among proteins in a pancreatic juice component-containing biological sample such as pancreatic or duodenal juice at room temperature; and a kit suited for this method and for storing a pancreatic juice component-containing biological sample at room temperature.

### Means for solving the Problems

With a view to overcoming the above-mentioned problem, the present inventors have carried out an intensive investigation. As a result, it has been found that by adding sulfonyl fluoride group-containing protease inhibitors and a trypsin-like serine protease inhibitor of a specific kind, as defined in the claims, in combination to a pancreatic juice component-containing biological sample, S100P contained in the biological sample can be stored stably even when the sample is stored at room
temperature, leading to the completion of the invention.

Described specifically, the method for preparing a pancreatic juice component-containing sample and a kit for storing a pancreatic juice component-containing sample at room temperature, each according to the present invention, have the following constitutions (1) to (5):
(1) A method for preparing a pancreatic juice component-containing sample, comprising a step of adding, to a pancreatic juice component-containing biological sample, PMSF and AEBSF as a sulfonyl fluoride group-containing protease inhibitor and TLCK as a trypsin-like serine protease inhibitor belonging to a group of amino acid chloromethyl ketones, thereby preparing the pancreatic juice component-containing sample.
(2) The method for preparing a pancreatic juice component-containing sample according to above (1), further comprising, after the step of preparing the pancreatic juice component-containing sample, storing the pancreatic juice component-containing sample at room temperature.
(3) The method for preparing a pancreatic juice component-containing sample according to above (1), wherein in the step of preparing the pancreatic juice component-containing sample, PMSF, and AEBSF are added as the sulfonyl fluoride group-containing protease inhibitor to give a final concentration of 1 mM or more, and 4 mM or more, respectively and TLCK is added as the trypsin-like serine protease inhibitor to give a final concentration of 0.1 mM or more.
(4) The method for preparing a pancreatic juice component-containing sample according to above (1), wherein the biological sample is a pancreatic juice or a duodenal juice.
(5) A kit for storing a pancreatic juice component-containing biological sample at room temperature, comprising:
   a storage container equipped with a reservoir portion for retaining therein a collected body fluid,
   the reservoir portion being filled in advance with PMSF and AEBSF as a sulfonyl fluoride group-containing protease inhibitor and
   TLCK as a trypsin-like serine protease inhibitor.

### Effects of the Invention

The method for preparing a pancreatic juice component-containing sample according to the present invention makes it possible to prepare a pancreatic juice component-containing biological sample, that is a storage sample that can be stored stably at room temperature while sufficiently suppressing degradation or modification of S100P contained in the biological sample. This means that the method makes it possible to stably store an easily degradable protein in pancreatic juice, thereby provide a high quality sample and moreover, this method is expected to contribute to acceleration of the study of pancreatic cancer. In particular, the method for preparing a pancreatic juice component-containing sample according to the present invention enables preparation of a sample that can be stored at room temperature so that it is expected to be applied widely to clinical tests.

In addition, the kit for storing a pancreatic juice component-containing biological sample at room temperature according to the present invention makes it possible to more simply and easily prepare a biological sample for the S100P test that can be stored at room temperature by using pancreatic or duodenal juice collected from a living body.

### Brief Description of the Drawings

FIG. 1 shows chemical formulas of PMSF, AEBSF, and p-APMSF, respectively.
FIG. 2 shows the results of the protease activity (relative value: %) of respective pancreatin solutions in Referential Example 1.
FIG. 3 shows the measurement results of the amount of S100P in the respective sample solutions in Referential Example 2.
FIG. 4 shows the measurement results of the amount of S100P in the respective sample solutions in Example 1.
FIG. 5 shows the measurement results of the amount of S100P in the respective sample solutions in Referential Example 3.

### Embodiments for carrying out the Invention

Pancreatic juice is a body fluid excreted from the pancreatic duct. The term "pancreatic juice component-containing biological sample" as used in the present invention and present specification means a sample containing a body fluid containing a pancreatic juice derived component. Examples of the body fluid containing a pancreatic juice derived component include pancreatic juice collected from the pancreas directly through a catheter and a fluid collected in the duodenum (duodenal juice). The duodenal juice contains, in addition to the pancreatic juice, bile excreted from the papillary portion and a fluid, blood, and the like originally present in the duodenum. The pancreatic juice or duodenal juice can be collected in a manner known per se in the art.

### <Method for preparing a pancreatic juice component-containing sample>

The method for preparing a pancreatic juice component-containing sample according to the present invention (which may hereinafter be called "preparation method of the present invention is a method for preparing a pancreatic juice component-containing biological sample to permit stable storage of it even at room temperature while suppressing degradation or modification of particularly S100P among components contained in the biological sample. Described specifically, the preparation method of the present invention is characterized in that it has a step of adding, to a pancreatic juice component-containing biological sample, at least two sulfonyl fluoride group-containing protease inhibitors and at least one trypsin-like serine protease inhibitor belonging to a group of amino acid chloromethyl ketones, as defined in the claims, to prepare a pancreatic juice component-containing sample and in addition, it has, if necessary, a step of storing the sample at room temperature until the sample is subjected to detection of S100P.

The pancreatic juice component-containing biological sample such as pancreatic juice or duodenal juice contains a large amount of proteases as well as proteins to be tested (S100P in the preparation method of the present invention) therefore, when the biological sample is stored at room temperature, the target protein is inevitably degraded or modified due to the influence of the proteases. When the biological sample after stored at room temperature is tested, therefore, the protein is not detected at the test even from
the biological sample which contained the protein immediately after collection from a living body, causing a problem of lower accuracy of the test. On the other hand, when a pancreatic juice component-containing biological sample is prepared by using the preparation method of the present invention, the activity of the proteases derived from the biological sample can be effectively suppressed and stability of S100P can be improved drastically even if the sample is in solution form at room temperature. That is, the pancreatic juice component-containing sample obtained using the preparation method of the present invention has a quality higher than ever even after storage at room temperature and it is therefore suited for the S100P test (test for detecting S100P) requiring high accuracy.

More specifically, in the preparation method of the present invention, the pancreatic juice component-containing sample is prepared by adding at least two sulfonyl fluoride group-containing protease inhibitors and at least one trypsin-like serine protease inhibitor belonging to a group of amino acid chloromethyl ketones (which may hereinafter be called "specific trypsin-like serine protease inhibitor"), as defined in the claims. By using the sulfonyl fluoride group-containing protease inhibitor and the trypsin-like serine protease inhibitor in combination, degradation or modification of S100P can effectively be suppressed even though it is in a solution at room temperature.

As described later in Referential Example 1, the effect of amino acid chloromethyl ketones such as TLCK (N-a-tosyl-L-lysine chloromethyl ketone) having an inhibitory activity against a trypsin-like serine protease for suppressing the activity of proteases in pancreatic or duodenal juice is only the same level as that of Foipan or FOY and is much lower than that of another generally used protease inhibitor such as leupeptin. Using a sulfonyl fluoride group-containing protease inhibitor and a trypsin-like serine protease inhibitor belonging to a group of amino acid chloromethyl ketones such as TLCK in combination, however, has produced such an excellent effect of improving the room temperature stability of S100P as cannot be observed when a sulfonyl fluoride group-containing protease inhibitor and another protease inhibitor such as leupeptin were used in combination. This astonishing finding that use of a sulfonyl fluoride group-containing protease inhibitor and a trypsin-like serine protease inhibitor belonging to a group of amino acid chloromethyl ketones in combination is particularly effective for the improvement of stability of S100P at room temperature has been found for the first time by the present inventors.

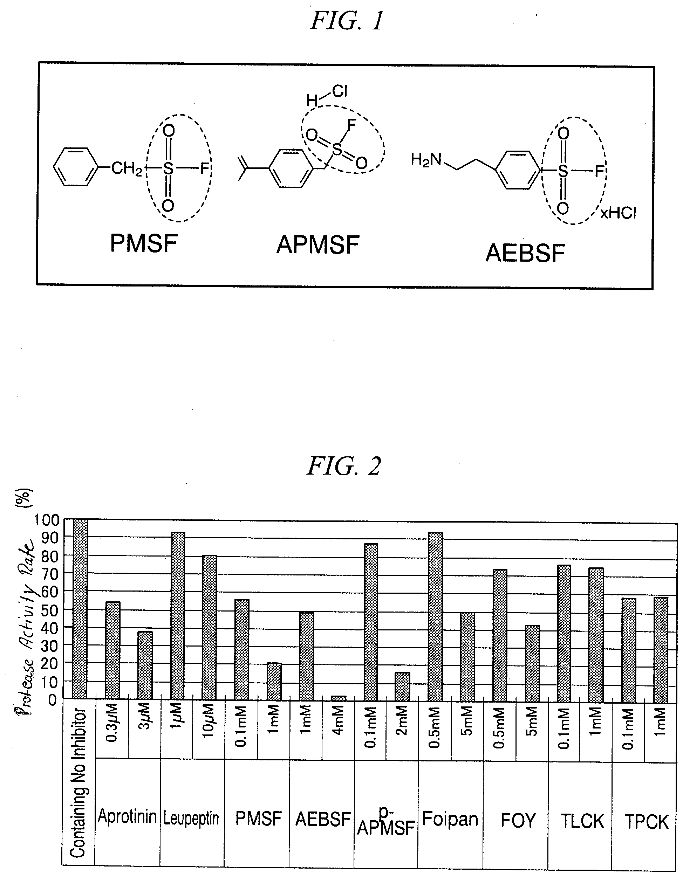

The sulfonyl fluoride group-containing protease inhibitors to be used in the preparation method of the present invention is
a compound having a sulfonyl fluoride group and at the same time, having protease inhibitory activity, , more specifically PMSF (phenylmethylsulfonyl fluoride), and AEBSF (4-(2-aminoethyl)-benzenesulfonyl fluoride)). Further sulfonyl fluoride group-containing protease inhibitors are p-APMSF (p-amidinophenylmethanesulfonyl fluoride hydrochloride), 4-(fluorosulfonyl)benzoic acid (CAS No. 455-26-5), 3-(fluorosulfonyl)benzoic acid (CAS No. 454-95-5), 2-aminobenzenesulfonyl fluoride (CAS No. 392-86-9), 3-aminobenzenesulfonyl fluoride (CAS No. 368-50-3), 4-aminobenzenesulfonyl fluoride (CAS No. 98-62-4), 2-nitrobenzenesulfonyl fluoride (CAS No. 433-98-7), 3-nitrobenzenesulfonyl fluoride (CAS No. 349-78-0), or 4-nitrobenzenesulfonyl fluoride (CAS No. 349-96-2). The chemical formulas of PMSF, AEBSF, and p-APMSF are shown in FIG. 1, respectively. The functional group surrounded with a dotted line in FIG. 1 is a sulfonyl fluoride group.

The sulfonyl fluoride group-containing protease inhibitors to be used in the preparation method of the present invention are used in combination of two or more. Using at least PMSF and AEBSF is preferred and using
PMSF, AEBSF, and p-APMSF in combination is more preferred. All of these three compounds may be used. Since pancreatic juice contains various proteases different in activity, using a plurality of protease inhibitors as a mixture is expected to reliably suppress the protease activity in a biological sample. Further, using a plurality of them in combination at a low concentration is expected to suppress the protease activity compared with single use.

The amount of the sulfonyl fluoride group-containing protease inhibitor to be added to the pancreatic juice component-containing biological sample is not particularly limited as long as it is an amount at which the protease inhibitor can produce a protease inhibiting effect and an effect of improving the room temperature stability of S100P. It can be adjusted as needed in consideration of the kind of the pancreatic juice component-containing biological sample, the kind of the protease inhibitor to be used, and the like. For example, when only PMSF is added as the sulfonyl fluoride group-containing protease inhibitor to the pancreatic juice component-containing biological sample, it can be added to give a final concentration of 1 mM or more, preferably 5 mM or more, more preferably 10 mM or more. For example, when only AEBSF is used as the sulfonyl fluoride group-containing protease inhibitor, AEBSF can be added to the pancreatic juice component-containing biological sample to give a final concentration of 4 mM or more, preferably 10 mM or more, more
preferably 20 mM or more. For example, when only p-APMSF is added as the sulfonyl fluoride group-containing protease inhibitor, p-APMSF can be added to the pancreatic juice component-containing biological sample to give a final concentration of 2 mM or more, preferably 5 mM or more, more preferably 10 mM or more.

The trypsin-like serine protease inhibitor to be used in the preparation method of the present invention is a compound that belongs to a group of amino acid chloromethyl ketones and has inhibitory activity against trypsin-like serine proteases typified by trypsin. In the present invention, TLCK is used.

The amount of the specific trypsin-like serine protease inhibitor to be added to the pancreatic juice component-containing biological sample is not particularly limited as long as it is an amount at which this protease inhibitor can produce a protease inhibitory effect and an effect of improving room temperature stability of S100P. It can be adjusted as needed in consideration of the kind of the pancreatic juice component-containing biological sample, the kind of the protease inhibitor to be used, and the like. For example, only TLCK is added as the specific trypsin-like serine protease inhibitor to the pancreatic juice component-containing biological sample, it can be added to give a final
concentration of 0.1 mM or more, preferably 1 mM or more, more preferably 5 mM or more, still more preferably 10 mM or more.

In the preparation method of the present invention, each of the sulfonyl fluoride group-containing protease inhibitor and the specific trypsin-like serine protease inhibitor to be added to the pancreatic juice component-containing biological sample may be either in solid form such as powders or granules or in the form of a protease inhibitor solution obtained by dissolving it in a proper buffer or the like. The sulfonyl fluoride group-containing protease inhibitor and the specific trypsin-like serine protease inhibitor may be added to the pancreatic juice component-containing biological sample simultaneously or after addition of one of them, the other one may be added. From the standpoint of sufficiently producing the effect of the present invention for improving the room stability of S100P, simultaneous addition of the sulfonyl fluoride group-containing protease inhibitor and the specific trypsin-like serine protease inhibitor is preferred.

In the preparation method of the present invention, only the sulfonyl fluoride group-containing protease inhibitor and the specific trypsin-like serine protease inhibitor may be added to the pancreatic juice component-containing biological sample or another protease inhibitor may be added further. No particular limitation is imposed on the another protease inhibitor as long as it does not spoil the effect of the invention for improving the room temperature stability of S100P. Examples of it include peptide-based protease inhibitors such as aprotinin, leupeptin, antipain, chymostatin, elastatinal, and antithrombin, chelating agents such as EDTA, elastase inhibitors, trypsin inhibitors, and Ecotin (E. coli). In addition, drugs for pancreatitis such as gabexate mesilate (FOY), camostat mesilate (Foipan), nafamostat mesilate (Futhan), and urinastatin can also be used.

The pancreatic juice component-containing biological sample to be provided for use in the preparation method of the present invention is not limited as long as it may be a sample containing a body fluid containing a pancreatic juice-derived component. It may be any of a sample composed only of a body fluid containing a pancreatic juice-derived component, a liquid obtained by diluting the body liquid with a proper buffer or the like, and a mixture obtained by adding, to the body liquid or diluted liquid, various additives. Examples of the additives include surfactants, nucleolytic enzyme inhibitors, pH regulators, and pH indicators. As the pancreatic juice component-containing biological sample to be provided for use in the preparation method of the present invention is preferably a sample containing pancreatic juice or duodenal juice. Specific examples include pancreatic juice, duodenal juice, diluted pancreatic juice, and diluted duodenal juice, and mixtures obtained by adding thereto the above-mentioned various additives.

In the preparation method of the present invention, the sulfonyl fluoride group-containing protease inhibitors and the trypsin-like serine protease inhibitor belonging to a group of amino acid chloromethyl ketones may be added to the pancreatic juice component-containing biological sample stored and the like after collection from a living body. The time interval from collection of a pancreatic juice component-containing body liquid from a living body to addition thereto of these protease inhibitors is preferably as short as possible, with addition of these protease inhibitors to the pancreatic juice or duodenal juice immediately after collected from a living body being particularly preferred. In the pancreatic juice-containing biological sample, proteases contained therein have very strong action and proteolysis proceeds immediately after collection so that it is preferred to shorten the time lag between collection of the biological sample and mixing with the protease inhibitors. For example, by preliminarily filling a reservoir portion (a container-like member for retaining a collected body fluid therein) provided in a collection tool for collecting pancreatic juice or duodenal juice from a living body with the sulfonyl fluoride group-containing protease inhibitors and the trypsin-like serine
protease inhibitor belonging to a group of amino acid chloromethyl ketones, the pancreatic juice or duodenal juice collected from the living body can be mixed with the protease inhibitors in a collection container immediately. When the collection tool is not equipped with a reservoir portion and a storage container serving as a reservoir portion is connected to the collection tool, the storage container is preliminarily filled with the sulfonyl fluoride group-containing protease inhibitor and the trypsin-like serine protease inhibitor belonging to a group of amino acid chloromethyl ketones.

In the preparation method of the present invention, since the optimum combination of the protease inhibitors is used in order to suppress degradation or modification of particularly S100P among pancreatic juice-derived components, S100P contained in the pancreatic juice component-containing sample prepared by this preparation method can be stored more stably than ever before even at room temperature. It is therefore possible to carry out the S100P test for the pancreatic juice component-containing sample prepared by the preparation method of the present invention with sufficient test accuracy even when the pancreatic juice component-containing sample is stored at room temperature until it is subjected to the S100P test. The pancreatic juice component-containing sample prepared by the preparation method of the present invention can produce its effect fully when stored at room temperature, but it may be stored in frozen or refrigerated form.

The S100P test to be made for the pancreatic juice component-containing sample prepared by the preparation method of the present invention is not particularly limited as long as it is a test for detecting the S100P or determining the amount thereof. The S100P can be detected by various protein analyses using ELISA, immunochromatography, two-dimensional electrophoresis, western blotting, or mass analysis, various nucleic acid analyses using PCR, RT-PCR, or hybridization with a probe, and cell analyses such as counting of the cell number a cytology.

The preparation method of the present invention is expected to improve also the room temperature stability of a pancreatic juice-derived component other than S100P. The pancreatic juice component-containing sample prepared by the preparation method of the present invention can therefore be used also as a measurement sample for various tests of a component, other than S100P, in the pancreatic juice component-containing sample. A substance to be tested is not particularly limited as long as it is a biological component expected to be contained in pancreatic or duodenal juice. It may be a protein, it may be a nucleic acid such as DNA or RNA, or it may be a cell. For example, the pancreatic juice component-containing sample can be used for various protein analyses using ELISA, immunochromatography, two-dimensional electrophoresis, western blotting, or mass analysis, various nucleic acid analyses using PCR, RT-PCR, or hybridization with a probe, and cell analyses such as counting of the cell number and cytology.

### <Kit for storing a pancreatic juice component-containing biological sample>

The kit for storing a pancreatic juice component-containing biological sample according to the present invention (which may hereinafter be called "storage kit of the present invention") is characterized in that it has at least two sulfonyl fluoride group-containing protease inhibitors and at least one trypsin-like serine protease inhibitor belonging to a group of amino acid chloromethyl ketones, as defined in the claims. By adding sulfonyl fluoride group-containing protease inhibitors and a specific trypsin-like serine protease inhibitor which the storage kit has to a pancreatic juice component-containing biological sample, a pancreatic juice component-containing sample which can be stored at room temperature until it is provided for the detection of S100P can be prepared more simply and easily.

The sulfonyl fluoride group-containing protease inhibitors to be included in the storage kit of the present invention are used in combination. In the present invention, using at least
PMSF and AEBSF, is preferred, with additionally using p-APMSF being more preferred. Similarly, the trypsin-like serine protease inhibitors to be included in the storage kit of the present invention may be used either singly or in combination of two or more. In the present invention, TLCK is used as the trypsin-like serine protease inhibitor.

The various protease inhibitors included in the storage kit of the present invention may be in the form of lyophilized powders or in the form of tablets, granules, or the like formed from the lyophilized powders together with a proper excipient and the like. They may be provided in the form of a solution of a protease inhibitor dissolved in a proper buffer.

The storage kit of the present invention may further contain a buffer for diluting a collected body fluid, another protease inhibitor, a surfactant, a pH regulator, a pH indicator, or the like. Various additives such as surfactant, pH regulator, and pH indicator may be dissolved in a diluting buffer in advance. Further, the storage kit of the present invention may include, in order to permit dropwise addition of a predetermined amount of a prepared pancreatic juice component-containing sample (obtained by adding, to a pancreatic juice component-containing biological sample
various protease inhibitors included in the storage kit of the present invention and then adding, if necessary, another component) from a container filled with the pancreatic juice component-containing sample, a cap that can be attached to an opening portion of the container.

The storage kit of the present invention may include a storage container equipped with a reservoir portion for retaining therein a body fluid, such as pancreatic juice or duodenal juice, collected from a living body. The protease inhibitors of the present invention are contained in the reservoir portion in advance. When the storage container has been graduated, the amount of the biological sample charged in the storage container (a total amount of the biological sample and the protease inhibitors when the storage container is filled with the protease inhibitors in advance) can be confirmed visually and further, a final concentration of the protease inhibitors can be found at a glance.

It is the common practice to collect the pancreatic juice component-containing biological sample transendoscopically. As a constituent of the storage kit of the present invention, the kit may include a collecting tool for transendoscopically collecting the pancreatic juice component-containing biological sample. Examples of the collecting tool include a combination of a catheter that can
be inserted into an endoscopic device and a syringe and a probe equipped, at the end thereof, with an absorber that can be inserted into an endoscopic device. Examples of the catheter that can be inserted into an endoscopic device include a specimen collecting cube described in Japanese Patent Application, First Publication No. 2011-5009. These collecting tools preferably have a reservoir portion in which the protease inhibitors have been dispensed in advance. When the reservoir portion is removable, it can also be used as a storage container.

### Examples

The present invention will hereinafter be described in further detail by Examples and the like but the present invention is not limited to or by the following Examples.

### [Referential Example 1]

Among digestive enzymes in pancreatic juice, serine protease is known as an enzyme serving to trigger the degradation cascade of them. First, screening of a serine protease inhibitor having a high inhibitory effect against proteases contained in pancreatic juice was therefore performed.

More specifically, an inhibitor targeting a serine protease and having a high inhibitory effect against pancreatic juice was screened from commercially available protease inhibitors. The protease inhibitors used were aprotinin (product of Roche), leupeptin (product of Roche), PMSF (product of Roche), AEBSF (product of Roche), p-APMSF (product of SIGMA), camostat mesilate (Foipan: product of Wako Pure Chemical Industries), gabexate mesilate (FOY: product of Wako Pure Chemical Industries), TLCK (product of SIGMA), and TPCK (product of SIGMA). It is to be noted that TPCK is an amino acid chloromethyl ketone having inhibitory activity against chymotrypsin but having no inhibitory activity against trypsin.

"Pancreatin", a digestive enzyme prepared from the pig pancreas in which a pancreatic enzyme was present in activated form was used as pseudo artificial pancreatic juice. After addition of protease inhibitors in an amount so that the final concentration thereof would fall within a concentration range recommended by the manufacturers of the inhibitors, the protease activity was measured. The protease activity was measured using EnzCheck Protease Assay Kits (product of Molecular Probes). Described specifically, fluorescently-labeled casein attached to a kit was added to a pancreatin solution containing each protease inhibitor. After incubation at 37°C for 2 hours, the amount of fluorescence was measured at a fluorescence wavelength of Ex/Em = 485/535 nm. As a control, the protease activity of a sample solution (containing no inhibitor) obtained by directly adding fluorescently-labeled casein to pancreatin was measured.

Measurement results of the amount of fluorescence of each of the pancreatin solutions are shown in FIG. 2. The amount of fluorescence plotted along the ordinate represents the amount of degraded casein and it was approximated as the value of protease activity. The protease inhibitors contained respectively in the pancreatin solutions and final concentrations thereof are plotted along the abscissa. The results have suggested that PMSF, AEBSF, and p-APMSF have a markedly high inhibitory effect compared with the other protease inhibitors. These inhibitors are each a sulfonyl fluoride group-containing sulfone-based compound.

### [Referential Example 2]

While attention was paid to the sulfone-based compounds found to have a high inhibitory effect against digestive enzymes in pancreatic juice in Referential Example 1, the effect of the various sulfone-based compounds on the room temperature stability of S100P (calcium-binding protein) known as a marker protein of pancreatic cancer was studied. That is, by using four human clinical pancreatic juice specimens, whether the concentration of S100P changed or not after addition of a S100P protein standard preparation was studied before and after room temperature storage.

Described specifically, prepared were sample solutions (Sample Solutions 1: containing no inhibitor) obtained by mixing 25 ng/mL of an S100P (standard preparation) solution
with the human clinical specimens, respectively; sample solutions (Sample Solutions 2: AEBSF) obtained by adding AEBSF (final concentration: 4 mM) to Sample Solutions 1, respectively; and sample solutions (Sample Solutions 3: AEBSF + PMSF) obtained by adding both AEBSF (final concentration: 4 mM) and PMSF (final concentration: 1 mM) to Sample Solutions 1, respectively. The sample solutions were each prepared using a buffer attached to CircuLex S100P ELISA Kit (product of Cyclex, Catalogue Number: CY-8060). The sample solutions thus obtained were each allowed to react by incubating at 25°C for 16 hours (room temperature storage). Then, S100P was detected using CircuLex S100P ELISA Kit (product of Cyclex, Catalogue Number: CY-8060) from solutions obtained by carrying out 10-fold dilution of the sample solutions with a buffer attached to CircuLex S100P ELISA Kit, respectively. As a control, S100P was detected similarly from Sample Solutions 1 immediately after preparation (that is, before room temperature storage).

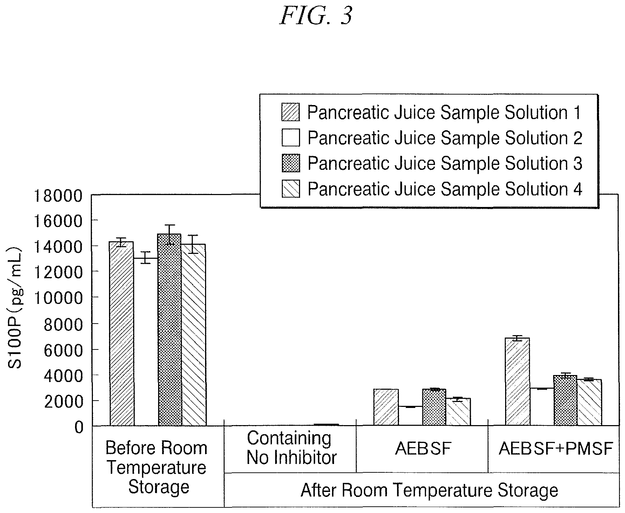

The measurement results are shown in FIG. 3. The S100P concentration is plotted along the ordinate, while Sample Solutions 1 before room temperature storage ("Before room temperature storage" in this graph) and Sample Solutions 1 to 3 after room temperature storage ("Containing no inhibitor", "AEBSF", and "AEBSF + PMSF" in this graph) are plotted along the abscissa. In Sample Solutions 1 containing no protease inhibitor, S100P of any of the pancreatic juice specimens was almost degraded after room temperature storage. On the other hand, in Sample Solutions 2 and 3 containing the protease inhibitor, S100P was detected from any of the pancreatic juice specimens. In particular, in Sample Solution 3 containing both AEBSF and PMSF, the S100P concentration was maintained high. The above-mentioned results have revealed that mixing of at least one, preferably two or more sulfonyl fluoride-containing sulfone-based compounds improves the stability of S100P in pancreatic juice even stored at room temperature.

### [Example 1]

In order to detect not an artificially added standard preparation but S100P originally contained in a human pancreatic juice specimen, addition of only a sulfonyl fluoride group-containing sulfone-based compound is insufficient. With a view to improving the storage performance of S100P further, it was studied whether the storage stability of S100P was influenced or not by the combined use of the sulfonyl fluoride group-containing sulfone-based compound and TLCK, that is, another sulfone-based compound. Seven human clinical specimens were used.

Described specifically, prepared were sample solutions (Sample Solutions 1: AEBSF) obtained by adding AEBSF (final concentration: 4 mM) to the human clinical specimens, respectively; sample solutions (Sample Solutions 2: AEBSF + PMSF) obtained by adding AEBSF (final concentration: 4 mM) and PMSF (final concentration: 1 mM) to the human clinical specimens, respectively; sample solutions (Sample Solutions 3: AEBSF + PMSF + Aprotinin) obtained by adding AEBSF (final concentration: 4 mM), PMSF (final concentration: 1 mM) and Aprotinin (final concentration: 3 µM) to the human clinical specimens, respectively; sample solutions (Sample Solutions 4: AEBSF + PMSF + TPCK) obtained by adding AEBSF (final concentration: 4 mM), PMSF (final concentration: 1 mM), and TPCK (final concentration: 1 mM) to the human clinical specimens, respectively; and sample solutions (Sample Solutions 5: AEBSF + PMSF + TLCK) obtained by adding AEBSF (final concentration: 4 mM), PMSF (final concentration: 1 mM), and TLCK (final concentration: 1 mM) to the human clinical specimens, respectively. The sample solutions were each prepared using a buffer attached to CircuLex S100P ELISA Kit (product of Cyclex Inc., Catalog number: CY-8060). After those sample solutions were each allowed to react by incubating them at 25°C for 16 hours (after storage at room temperature), detection of S100P was performed in a manner similar to that of Referential Example 2.

As a control, Sample Solutions 1 to 5 were prepared in a manner similar to that described above except freeze storage was performed instead of room temperature storage and detection of S100P from the sample solutions was performed in a manner similar to that of Referential Example 2.

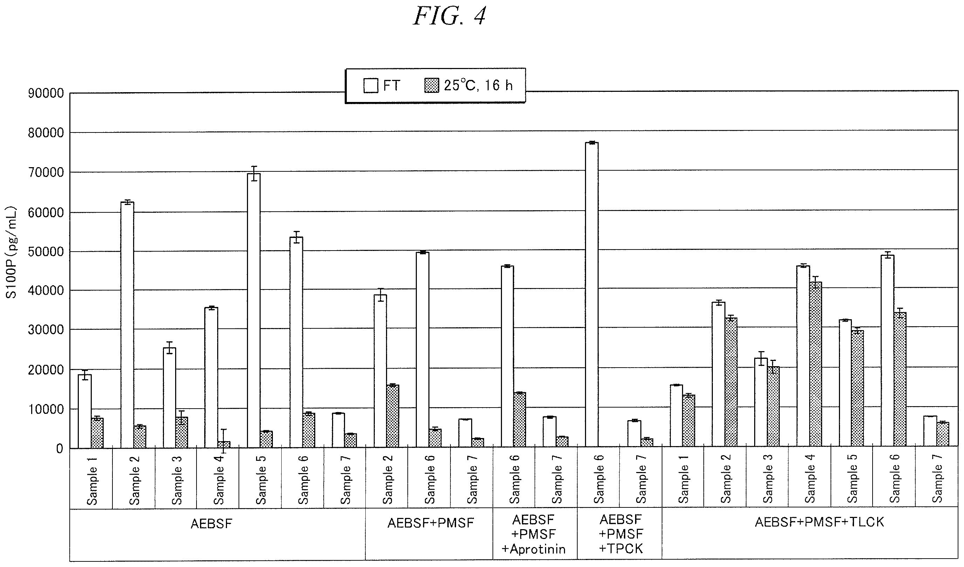

Measurement results are shown in FIG. 4. The concentration of S100P is plotted along the ordinate, while Sample Solutions 1 to 5 ("AEBSF", "AEBSF + PMSF", "AEBSF + PMSF + Aprotinin", "AEBSF + PMSF + TPCK", and "AEBSF + PMSF + TLCK" in the graph) of the human pancreatic juice specimens 1 to 7 are plotted along the abscissa. The term "FT" in the graph shows the results after freeze storage without room temperature storage and the term "25°C, 16h" shows the results after incubation at 25°C for 16 hours (after room temperature storage), respectively. As a result, in the sample solutions after freeze storage, S100P was detected, though varying in amount, from all the human pancreatic juice specimens irrespective of the kind of the protease inhibitor added. On the other hand, in Sample solutions 1 containing only AEBSF or Sample Solutions 2 containing both AEBSF and PMSF, S100P was detected from all the human pancreatic juice specimens, but the detection amount was very small compared with that after freeze storage. It has revealed that during room temperature storage, most of S100P was degraded or modified. In Sample Solutions 5 containing AEBSF, PMSF, and TLCK, on the other hand, all the human pancreatic juice specimens showed an S100P concentration remaining ratio as high as about 70% or more of that after freeze storage. In Sample Solutions 3 containing AEBSF, PMSF, and Aprotinin or Sample Solutions 4 containing AEBSF, PMSF, and TPCK, the remaining ratio was not so high as that of Sample Solutions 5 containing AEBSF, PMSF, and TLCK. The above-mentioned results have revealed that by using at least one sulfonyl fluoride group-containing protease inhibitor and an amino acid chloromethyl ketone having trypsin-like serine protease inhibitory activity such as TLCK in combination, S100P in pancreatic juice can be stored very stably even at room temperature.

### [Referential Example 3]

An effect of a protease inhibitor for storage stability of S100P in a pseudo artificial pancreatic juice and that for storage stability of S100P in a human clinical specimen were compared. As a S100P-containing pseudo artificial pancreatic juice, a solution obtained by adding S100P (standard preparation) to a pancreatin solution to give a concentration of 25 ng/mL was used.

More specifically, prepared were sample solutions (Sample Solutions 1: containing no inhibitor) obtained by mixing 25 ng/mL of an S100P (standard preparation) solution with the two human clinical specimens (human pancreatic juice specimens 1 and 2) used in Example 1 and the pseudo artificial pancreatic juice, respectively; sample solutions (Sample Solutions 2: AEBSF) obtained by adding AEBSF (final concentration: 4 mM) to Sample Solutions 1, respectively; sample solutions (Sample Solutions 3: AEBSF + PMSF) obtained by adding AEBSF (final concentration: 4 mM) and PMSF (final concentration: 1 mM) to Sample Solutions 1, respectively; and sample solutions (Sample Solutions 4: AEBSF + PMSF + TLCK) obtained by adding AEBSF (final concentration: 4 mM), PMSF (final concentration: 1 mM) and TLCK (final concentration: 1 mM) to Sample Solutions 1, respectively. Each of the sample solutions was prepared using a buffer attached to CircuLex S100P ELISA Kit (product of Cyclex Inc., Catalog number: CY-8060). After the sample solutions were allowed to react by incubating them at 25°C for 16 hours (after storage at room temperature), detection of S100P was performed in a manner similar to that of Referential Example 2.

As a control, after Sample Solutions 1 prepared in a manner similar to that described above were subjected to freeze storage instead of room temperature storage, detection of S100P was performed in a manner similar to that of Referential Example 2.

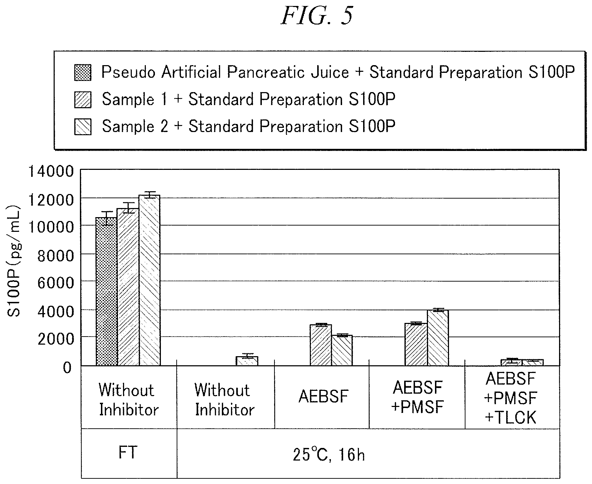

Measurement results are shown in FIG. 5. The concentration of S100P is plotted along the ordinate, while Sample Solutions 1 to 4 using the human pancreatic juice specimens 1 and 2 and the S100P-containing pseudo artificial pancreatic juice ("Without inhibitor", "AEBSF", "AEBSF + PMSF", and "AEBSF + PMSF + TLCK" in the graph) are plotted along the abscissa. The term "FT" in the graph shows the results after freeze storage without room temperature storage and the term "25°C, 16h" shows the results after incubation at 25°C for 16 hours (after room temperature storage), respectively. As a result, in the sample solutions after freeze storage, S100P was detected from both the human pancreatic juice specimen and the pancreatin solution. On
the other hand, with regard to the sample solutions after room temperature storage, no S100P was detected from the pancreatin solutions irrespective of the presence or absence or the kind of the protease inhibitor. With regard to the human pancreatic juice specimens 1 and 2, the detection amount of S100P is greater in Sample Solutions 2 and 3 containing AEBSF or AEBSF and PMSF than in Sample Solutions 1 containing no protease inhibitor, suggesting that the protease inhibitor added is effective for improving the room temperature stability of S100P. On the other hand, in both the human pancreatic juice specimens 1 and 2, different from the results of Example 1, the detection amount of S100P was obviously smaller in Sample Solutions 4 containing AEBSF, PMSF, and TLCK than in Sample Solutions 2 or 3 containing no TLCK. These results have revealed that, although the reason is not clear, a room temperature storage stability effect for S100P produced by using a sulfonyl fluoride group-containing protease inhibitor such as AEBSF and a trypsin-like serine protease inhibitor, such as TLCK, belonging to a group of amino acid chloromethyl ketones in combination is valid for S100P (endogenous S100P) originally contained in a biological sample such as pancreatic juice or duodenal juice collected from a living body but not valid for S100P (exogenous S100P) added later.

### Industrial Applicability

The preparation method of the present invention and the room temperature storage kit of the present invention enable stable storage of particularly S100P among proteins in biological samples such as pancreatic juice and duodenal juice at room temperature so that they can be used in the field of analyzing S100P in pancreatic juice, particularly in the field of clinical tests for diagnosis and treatment of pancreatic cancer.

## Claims

1. A method for preparing a pancreatic juice component-containing sample, comprising a step of adding, to a pancreatic juice component-containing biological sample, PMSF and AEBSF as a sulfonyl fluoride group-containing protease inhibitor and TLCK as a trypsin-like serine protease inhibitor belonging to a group of amino acid chloromethyl ketones, thereby preparing the pancreatic juice component-containing sample.

2. The method for preparing a pancreatic juice component-containing sample according to Claim 1, further comprising, after the step of preparing the pancreatic juice component-containing sample, storing the pancreatic juice component-containing sample at room temperature.

3. The method for preparing a pancreatic juice component-containing sample according to Claim 1, wherein in the step of preparing the pancreatic juice component-containing sample, PMSF, and AEBSF are added as the sulfonyl fluoride group-containing protease inhibitor to give a final concentration of 1 mM or more, and 4 mM or more, respectively and TLCK is added as the trypsin-like serine protease inhibitor to give a final concentration of 0.1 mM or more.

4. The method for preparing a pancreatic juice component-containing sample according to Claim 1, wherein the biological sample is a pancreatic juice or a duodenal juice.

5. A kit for storing a pancreatic juice component-containing biological sample at room temperature, comprising:
a storage container equipped with a reservoir portion for retaining therein a collected body fluid,
the reservoir portion being filled in advance with PMSF and AEBSF as a sulfonyl fluoride group-containing protease inhibitor and
TLCK as a trypsin-like serine protease inhibitor.

## Patentansprüche

1. Verfahren zum Herstellen einer Pankreassaftkomponente enthaltenden Probe, umfassend einen Schritt des Hinzufügens von PMSF und AEBSF als einen Sulfonylgruppe enthaltenden Proteaseinhibitor, und TLCK als einen trypsinartige Serinproteaseinhibitor, der einer Gruppe von Aminosäure-Chlormethylketonen angehört, zu einer Pankreassaftkomponente enthaltenden biologischen Probe, dadurch Herstellen der Pankreassaftkomponente enthaltenden Probe.

2. Verfahren zum Herstellen einer Pankreassaftkomponente enthaltenden Probe nach Anspruch 1, des Weiteren umfassend, nach dem Schritt des Herstellens der Pankreassaftkomponente enthaltenden Probe, Lagern der Pankreassaftkomponente enthaltenden Probe bei Raumtemperatur.

3. Verfahren zum Herstellen einer Pankreassaftkomponente enthaltenden Probe nach Anspruch 1, wobei in dem Schritt des Herstellens der Pankreassaftkomponente enthaltenden Probe PMSF und AEBSF als der Sulfonylgruppe enthaltende Proteaseinhibitor hinzugefügt werden, eine Endkonzentration von 1 mM oder mehr beziehungsweise 4 mM oder mehr zu ergeben, und TLCK als der trypsinartige Serinproteaseinhibitor hinzugefügt wird, eine Endkonzentration von 0,1 mM oder mehr zu ergeben.

4. Verfahren zum Herstellen einer Pankreassaftkomponente enthaltenden Probe nach Anspruch 1, wobei die biologische Probe ein Pankreassaft oder ein Duodenalsaft ist.

5. Ein Kit zum Lagern einer Pankreassaftkomponente enthaltenden biologischen Probe bei Raumtemperatur, umfassend:
einen mit einem Reservoirteil ausgestatteten Lagerbehälter zum Aufbewahren einer gesammelten Körperflüssigkeit darin,
wobei der Reservoirteil im Voraus mit PMSF und AEBSF als einen Sulfonylgruppe enthaltende Proteaseinhibitor und
TLCK als einen trypsinartige Serinproteaseinhibitor vorgefüllt ist.

## Revendications

1. Procédé de préparation d'un échantillon contenant un constituant de suc pancréatique, comprenant une étape d'ajout, à un échantillon biologique contenant un constituant de suc pancréatique, de PMSF et d'AEBSF en tant qu'inhibiteur de protéase contenant un groupe fluorure de sulfonyle et de TLCK en tant qu'inhibiteur de sérine-protéase analogue à la trypsine appartenant à un groupe d'acide aminé chlorométhylcétones, préparant ainsi l'échantillon contenant un constituant de suc pancréatique.

2. Procédé de préparation d'un échantillon contenant un constituant de suc pancréatique selon la revendication 1, comprenant en outre, après l'étape de préparation de l'échantillon contenant un constituant de suc pancréatique, le stockage de l'échantillon contenant un constituant de suc pancréatique à température ambiante.

3. Procédé de préparation d'un échantillon contenant un constituant de suc pancréatique selon la revendication 1, dans lequel, à l'étape de préparation de l'échantillon contenant un constituant de suc pancréatique, du PMSF et de l'AEBSF sont ajoutés en tant qu'inhibiteur de protéase contenant un groupe fluorure de sulfonyle pour donner une concentration finale de respectivement, 1 mM ou plus, et 4 mM ou plus, et de la TLCK est ajoutée en tant qu'inhibiteur de sérine-protéase analogue à la trypsine pour donner une concentration finale de 0,1 mM ou plus.

4. Procédé de préparation d'un échantillon contenant un constituant de suc pancréatique selon la revendication 1, dans lequel l'échantillon biologique est un suc pancréatique ou un suc duodénal.

5. Kit de stockage d'un échantillon biologique contenant un constituant de suc pancréatique à température ambiante, comprenant :
un récipient de stockage muni d'une partie réservoir pour retenir dans celle-ci un liquide organique collecté,
la partie réservoir étant remplie à l'avance de PMSF et d'AEBSF en tant qu'inhibiteur de protéase contenant un groupe fluorure de sulfonyle et
de la TLCK en tant qu'inhibiteur de sérine-protéase analogue à la trypsine.
